# EUROPEAN PATENT SPECIFICATION

(11) **EP 0 616 033 B1**
(45) Date of publication and mention of the grant of the patent: **13.06.2001**
(21) Application number: 93200773.5
(22) Date of filing: 17.03.1993
(51) Int. Cl.: C12N 15/57, C12N 9/54

(54) **Mutants of a thermostable neutral protease from Bacillus**
Mutanten von einer thermostabilen neutralen Protease aus Bacillus
Mutants d'une protéase neutre thermostable du Bacillus

(43) Date of publication of application: 21.09.1994
(73) Proprietor: HOLLAND SWEETENER COMPANY V.O.F., 6212 XW Maastricht (NL); SAGAMI CHEMICAL RESEARCH CENTER, Chiyoda-ku Tokyo 100 (JP)
(72) Inventor: Nagao, Hiromasa, Kanagawa-pref., Fujisawa-city (JP); Yoneya, Takashi, Tokyo-pref., Machida-city (JP); Miyake, Toshio, Kanagawa-pref., Yokohama-city (JP); Aoyama, Atsuo, Kanagawa-pref., Ebina-city (JP); Kai, Ken-ichi, Kanagawa-pref., Yamato-city (JP); Kidokoro, Shun-ichi, Kanagawa-pref., Sagamihara-city (JP); Miki, Yoichiro, Kanagawa-pref., Sagamihara-city (JP); Endo, Kimiko, Tokyo-pref., Machida-city (JP); Wada, Akiyoshi, Tokyo-pref., Minato-ku (JP)
(74) Representative: den Hartog, Jeroen Hendrikus Joseph

(56) References cited:
- NATURE vol. 324, no. 6098, 18 December 1986, LONDON GB pages 695 - 697 T. IMANAKA ET AL. 'A new way of enhancing the thermostability of proteases'
- APPLIED AND ENVIRONMENTAL MICROBIOLOGY vol. 58, no. 11, November 1992, US pages 3779 - 3783 M. KUBO ET AL. 'Alteration of specific activity and stability of thermostable neutral protease by site-directed mutagenesis - Bacillus stearothermophilus enzyme engineering for use in aspartame production'
- DATABASE WPI Section Ch, Week 9303, Derwent Publications Ltd., London, GB; Class B04, AN 93-022706
- BIOSIS PREVIEWS, BIOSIS, PHILADELPHIA, PA, US. ABSTRACT NO. 87130218 S.V. KOSTROV ET AL. 'Structure-function relationship for Bacilli metalloproteases'
- PROTEIN ENGINEERING vol. 5, no. 5, 1992, pages 421 - 426 V.G.H. EIJSINK ET AL. 'The effect of cavity-filling mutations on the thermostability of Bacillus stearothermophilus neutral protease'
- ANNALS OF THE NEW YORK ACADEMY SCIENCE vol. 613, 1990, NEW YORK, N.Y., US pages 347 - 351 T. IMANAKA 'Enhancement of thermostability of neutral proteases'

## Description

### FIELD OF THE INVENTION

This invention relates to novel thermolysin-like neutral metallo-proteases and to use thereof, more specifically in the production of benzyloxycarbonyl-α-L-aspartyl-L-phenylalanine methyl ester.

Thermolysin is a useful enzyme which is commercially available and used in a wide variety of fields, for example in detergent compositions, in food processing and in cosmetic formulations. It is further used in the synthesis of benzyloxycarbonyl-α-L-aspartyl-L-phenylalanine methyl ester (hereinafter referred to briefly as Z-APM), which is a precursor of aspartame, an artificial sweetener.

### BACKGROUND OF THE INVENTION

Thermolysin was first found in the culture broth of Bacillus thermoproteolyticus (Endo, S. (1962) J. Fermentation Tech., 40, 346-353) and a number of investigations have been conducted thereon. Thus, for instance, its amino acid sequence (Titani, K., et al., (1972) Nature New Biol., 238, 35-37) and the three-dimensional structure of the enzyme (Holmes, M.A. and Matthews, B.W., (1982) J. Mol. Biol. 160., 623-639) have been elucidated. Meanwhile, the protease gene was cloned from Bacillus thermoproteolyticus (EP-A-0418625) and the amino acid sequence of the mature enzyme as deduced frcm the nucleotide sequence of said gene was found to be different from the original primary structure as indicated by Titani in two positions. Thus, it was reported that the 37th (from the amino terminal) amino acid residue of the mature enzyme is not aspartic acid but asparagine and the 119th one is not glutamic acid but glutamine. This amino acid sequence is identical with that coded by nprM, one of the protease genes cloned from Bacillus stearothermophilus (Kubo, M., et al., (1988) Journal of General Microbiology 134, 1883-1892).

Therefore, in the present specification, the protease coded by this nprM gene or the gene from Bacillus thermoproteolyticus is referred to as "wild type thermolysin-like neutral metallo-protease".

Alteration of specific activity and stability of thermolysin-like neutral metallo-protease has very recently been reported (Kubo M., et al., (1992) Applied and Environmental Microbiology, 58, 3779-3783). In this article various mutants have been described which differ in one or more amino acid residues in the primary structure, especially at positions 93, 110, 114, 115, 136, 137, 143, 151, 157, 193, 211, 217 and 221. But in this reference, the activity was measured only by casein digestion method. None of these mutants, however, did show any substantially improved activity in relation to Z-APM synthesis or digestion. It now (as described further in the examples below) also has been established that the activity for casein digestion does not correlate to that for Z-APM synthesis: it appears that even if the specific activity for casein digestion increases, the specific activity for Z-APM synthesis does not always increase.

Based on these observations, and because there are various problems in the enzymatic synthesis of Z-APM, such as relatively low activity of the enzyme, inactivation of the enzyme during the condensation reaction and hydrolysis of the product Z-APM and the starting material L- or D,L-phenylalanine methyl ester (PM), due to the long reaction time, and/or unfavorable pH conditions, there is need to develop improved enzymes that have higher activity for the synthesis of Z-APM than wild-type thermolysin. Of course where PM is mentioned in this application also its salts are included in the meaning of the term PM.

### SUMMARY OF THE INVENTION

The object of the invention is to solve such problems as mentioned above. It was found that enzymes having excellent characteristics can be obtained when an appropriate substitute amino acid at certain positions is selected from among various candidates. Thus, from the thermolysin-like neutral metallo-protease having the (wild type) amino acid sequence of SEQ ID NO:1 shown below, novel proteases, having an improved activity in the synthesis of Z-APM in comparison with said wild type amino acid sequence, were derived by replacing one or more amino acid residues at certain positions in the sequence with other amino acid residues than the original ones. Specifically, the novel modified proteases according to the invention are obtained therefrom by replacement of at least one of the following amino acid residues with an amino acid different therefrom: 144th (leucine), 150th (aspartic acid), 187th (glutamic acid) and 227th (asparagine) amino acid residues and have an improved activity in the synthesis of Z-APM in comparison with the wild type metalls-protease having the amino acid sequence of SEQ ID NO:1.

In particular, the modified enzymes having the amino acid sequence as shown above, but with modification or of at least one of the amino acid residues at positions 144th, 150th, 187th and 227th, exhibit the higher specific activity for synthesis or digestion of Z-APM than the wild type enzyme. It solves the problems of how to enhance the activity of Z-APM synthesis of the thermolysin-like neutral metallo-protease derived from Bacillus stearothermophilus. Moreover, enzymes are provided which have higher activity at lower pH, and therefore ensure that during the synthesis of Z-APM less hydrolysis of Z-APM and PM occurs.

It is to be noted that those enzymes which are obtained by replacement at two or more of the positions specified above, naturally fall within the scope of the present invention. The same is true for those enzymes which are obtained by replacement of at least one of the amino acid residues specified above and by additional replacement of at least one of the other amino acid residues with an amino acid different therefrom.

### DETAILED DESCRIPTION OF THE INVENTION

Novel proteases described in this invention are derivatives having a modified sequence different from that of the wild type thermolysin-like neutral metallo-protease. In particular, the novel proteases have an enhanced activity for Z-APM synthesis and digestion. Typically, this is determined by analyzing the activity for Z-APM synthesis and/or digestion, and comparing these activities with that of wild type thermolysin-like neutral metallo-protease assayed in the same manner. This procedure is described further in the examples.

Modified enzymes can be produced by methods known per se to those skilled in the art.

The preferred method for producing the modified proteases of this invention is to introduce an amino acid sequence variation into predetermined positions of at least one of the 144th, 150th, 187th and 227th positions of the thermolysin-like neutral metallo-protease by recombinant DNA methods. The suitability of the proteases obtained can be determined by assay tests for the ultimate applications. Various methods are known which can be used to introduce mutations into cloned DNAs. For example, mutant nprM gene fragments are prepared by using the M13 phage mutagenesis method (Vandeyar, M., et al., (1988) Gene, 65, 129).

The plasmid and phage DNAs used for templates in this method, can be derived from the known plasmid pMK1 (Kubo, M. and Imanaka, T., (1989)J. Bacteriol, 171, 4080-4082). Several restriction endonucleases are used for digestion and cloning of the fragments of nprM gene into another plasmid or into phage vectors. The mutagenic primers should be complementary to the single-stranded template DNA containing the nprM gene, except for the codon(s) for the replaced amino acid residue(s). Various nucleotide sequences are conceivable for that purpose. By using these mutagenic primers which have (a) different codon(s) for the replaced amino acid residue(s), any desired amino acid replacement can be attained.

Alternatively, the nprM gene can be mutated by the PCR technique (polymerase chain reaction) using chemically synthesized primers (Higuchi, R., Krummel, B., and Saiki, R.K., (1988) Nucleic Acids Res. 16, 7351-7367). When a restriction enzyme site exists in the vicinity of the site of mutation, this PCR method is particularly useful. Since, for example, there is a cleavage site for the restriction enzyme SphI in the vicinity of the codon for aspartic acid in the 150th position of the wild type thermolysin-like metallo-protease, mutagenic primers containing this SphI site can be used for producing mutants in the 150th position. The mutagenic primer thus is used as a sense primer. As the reverse-direction primer (antisense), an oligonucleotide can be used, which is complementary to the nprM gene downstream from, for example, the AatI cleavage site of the nprM gene.

Two methods can be used for effecting mutagenesis at more than one site. One method comprises effecting simultaneous mutagenesis at all the target sites, while the other comprises introduction of mutations one after the other. Both methods actually give plasmids with mutations at more than one site.

A general method for recombinant thermolysin-like neutral metallo-protease preparation is described in the literature (Kubo, M. and Imanaka, T., (1989) J. Bacteriol., 171, 4080-4082) and comprises: insertion of the DNA encoding the modified thermolysin-like neutral metallo-protease into an expression vector, using this vector to transform a host cell, culturing the transformant until the modified metallo-protease accumulates in the culture and the then recovering modified enzyme from the culture. However, the plasmid pMK1 used in this reference is more than 20 kb in size and therefore it is substantially difficult to transform Escherichia coli with said plasmid. Furthermore, it was found that, in Bacillus subtilis too, the plasmid pMK1 drops out to a considerable extent in the latter stage of cultivation.

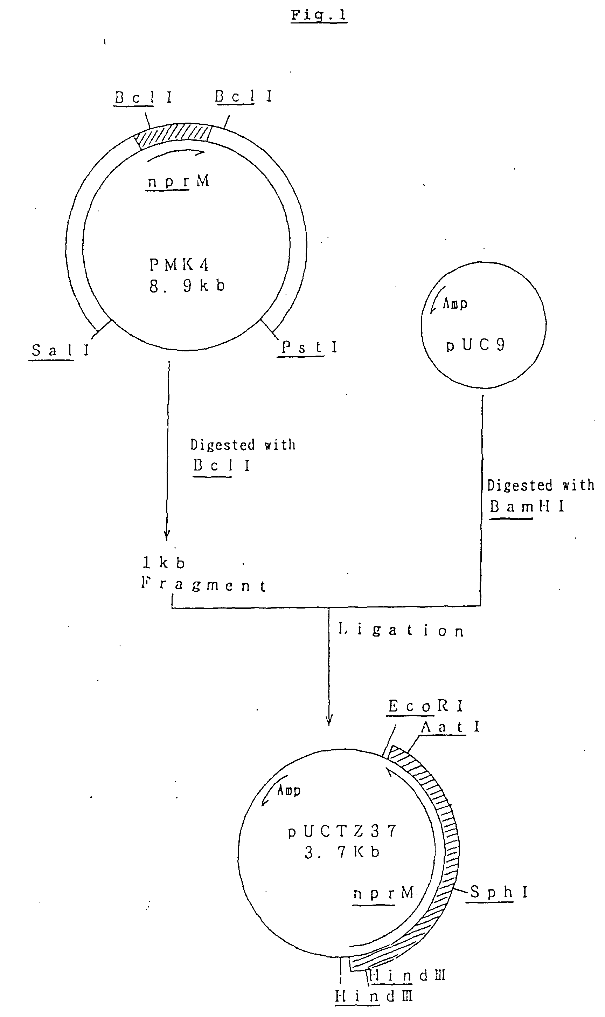

Therefore, to overcome such problems the inventors constructed shuttle vectors with which both hosts, Escherichia coli and Bacillus subtilis can be transformed and which can express the nprM gene in these hosts. As shown in Fig. 1 to Fig. 4, two shuttle vectors containing the nprM gene have been thus constructed (pUBTZ1 and pUBTZ2). When these are used to transform such strains of Escherichia coli as HB101 and JM103, the nprM gene is expressed in those strains. In addition, transformation of such Bacillus subtilis strains as DB104, DB117 and MT-2 with these plasmids led to successful expression of the nprM gene. Also no drop-out is observed in the latter stage of cultivation.

Similar results and advantages of using these shuttle vectors are obtained by using the modified thermolysin-like neutral metallo-protease genes instead of the wild type gene.

The modified thermolysin-like neutral metallo-protease can be produced in recombinant bacteria and is secreted in culture media. These proteases are recovered by ammonium sulfate precipitation and purified to homogeneity in the conventional manner, for example by hydrophobic interaction chromatography and/or gel filtration.

The modified proteases are used to synthesize Z-APM, which is a precursor of aspartame, more effectively than wild type thermolysin-like neutral metallo-protease. This is indicated by comparing the activities for the Z-APM digestion and Z-APM synthesis of these modified proteases to those of the wild-type enzyme. These are to be found extremely higher than those of the wild type enzyme. The measured values of these activities will be described in the following examples.

In this invention, the positions of the 144th, 150th, 187th and 227th from the amino-terminal amino acid in the thermolysin-like neutral metallo-protease have been found to have a great effect for enhancing the activity for Z-APM digestion and synthesis, especially when two or three of these sites were combined.

It is to be noted that the activity of casein digestion is not related to the activity of Z-APM synthesis or digestion. When the activities of mutant enzymes for casein and Z-APM are compared, it is clear that even if the activity for casein digestion is decreased, the activity for Z-APM synthesis and/or digestion can greatly be enhanced. The following examples are given only for illustrating the present invention and are by no means limitative of the scope of the invention.

### EXAMPLE 1

### (Synthesis of the mutant thermolysin-like neutral metallo-protease which has the mutation at the 187th (Example 1A) or 227th (Example 1B) amino acid residue.)

1 µg of the plasmid pMK1 containing the thermolysin-like neutral metallo-protease gene nprM derived from Bacillus stearothermophilus MK232 was digested with 5 units of each of PstI and BamHI in 20 µl of a reaction mixture (50 mM Tris-hydrochloride at pH 7.5, 10 mM MgCl₂, 1 mM DTT) at 37°C for 2 hours. The sample was subjected to 1% agarose gel electrophoresis, and an approximately 3.5 kb DNA fragment was separated and purified using a Bio-101 Gene Clean DNA purification kit.

Separately, 1 µg of the plasmid pUC9 was treated with 5 units of each of PstI and BamHI in 20 µl of the same reaction mixture as mentioned above at 37°C for 2 hours.

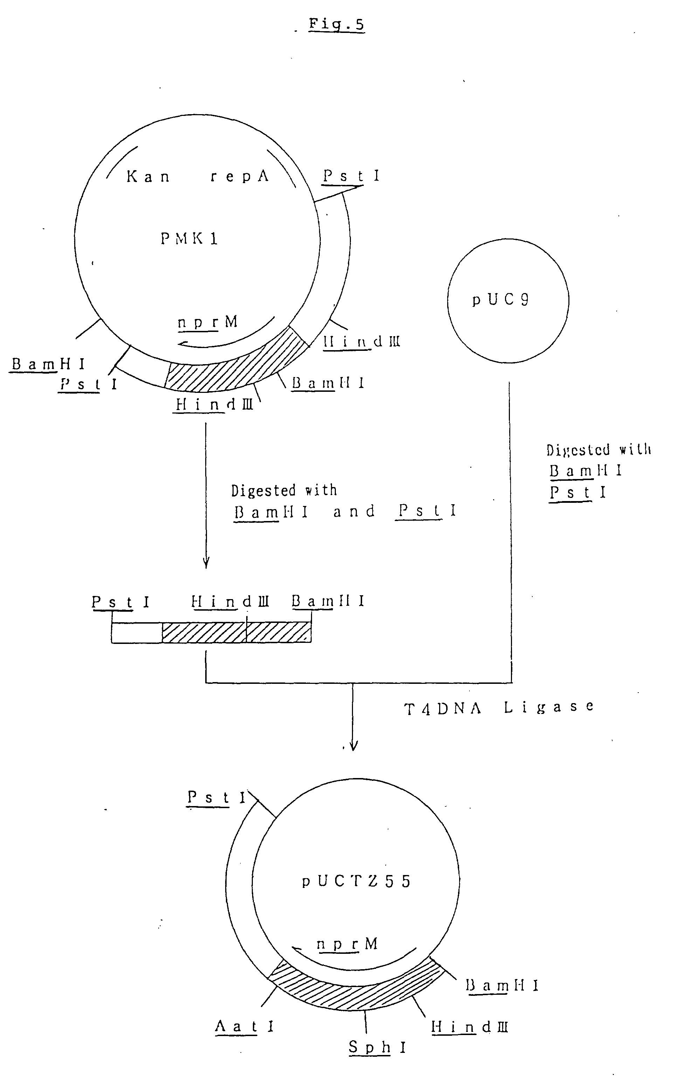

The PstI-BamHI fragment of the nprM gene was ligated with the PstI-BamHI digest of pUC9 using a Takara Shuzo DNA ligation kit. The ligation mixture was used to transform Escherichia coli JM109 in a conventional method to give a recombinant plasmid (pUCTZ55) containing the PstI-BamHI fragment of the nprM gene (Fig. 5).

1 µg of the recombinant plasmid pUCTZ55 was digested with 5 units of each of SphI and BclI in 20 µl of a reaction mixture (50 mM Tris-hydrochloride at pH 7.5, 10 mM MgCl₂, 100 mM NaCl, 1 mM DTT) at 37°C for 2 hours. The sample was subjected to 1% agarose gel electrophoresis and an approximately 550 kbp DNA fragment was separated and purified using a Bio-101 Gene Clean DNA purification kit.

Separately, 1 µg of the phage vector M13mp18 was digested with 5 units of each of SphI and BamHI in 20 µl of the same reaction mixture as mentioned above at 37°C for 2 hours.

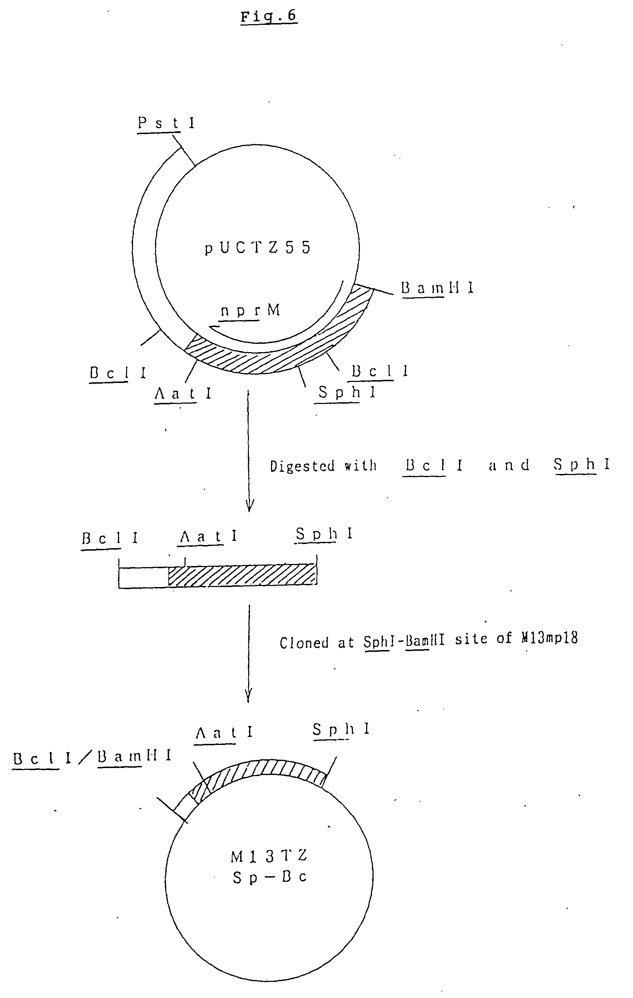

The SphI-BclI fragment of the nprM gene was ligated with the SphI-BamHI digest of M13mpl8 using a Takara Shuzo DNA ligation kit. The ligation mixture was used to transform Escherichia coli JM109 in a conventional method to give a recombinant phage (M13TZSp-Bc) containing the SphI-BclI fragment of the nprM gene (Fig. 6).

The single stranded DNA was prepared from the M13TZSp-Bc by a conventional method and subjected to mutagenesis. The oligonucleotides used for mutagenesis were prepared using an Applied Biosystems model 380B DNA synthesizer.

The mutagenic oligonucleotides used for the replacement of the 187th residue (glutamic acid to glutamine) (Example 1A) and the 227th residue (asparagine to histidine) (Example 1B) are shown below.

The mutageneses were performed using a USB T7-GEN in vitro mutagenesis kit, followed by DNA sequencing for confirmation of the mutation.

The double-stranded DNA of the mutated M13TZSp-Bc was prepared by a conventional method and 1 µg of the double-stranded DNA was digested with 5 units of each of SphI and AatI in 20 µl of reaction mixture (50 mM Tris-hydrochloride at pH 7.5, 10 mM MgCl₂, 100 mM NaCl, 1 mM DTT) at 37°C for 2 hours, and each sample was electrophoresed on a 1% agarose gel. A DNA fragment of about 550 kb was isolated from each of the M13TZSp-Bc digests and the DNA fragments were purified using a Bio-101 Gene Clean DNA purification kit.

Simultaneously, a vector fragment for the expression in Bacillus subtilis was produced by the method as described below. From a plasmid pMK4 (Yamada et al., (1991) Gene, 99, 109-114), the about 1.0 kb DNA fragment containing part of the nprM gene was digested with BclI and was cloned in BamHI site of a plasmid pUC9 to construct a plasmid pUCTZ37 (Fig. 1).

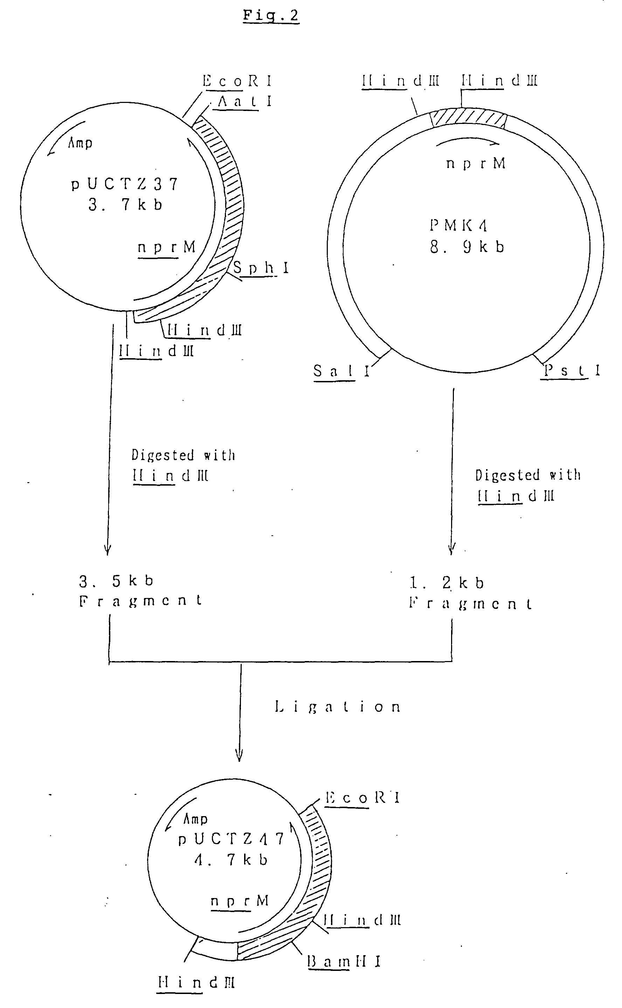

The plasmid pUCTZ37 was an incomplete one not having the 5'-terminal region of nprM gene. The plasmid pUCTZ37 was digested with restriction endonuclease HindIII and the about 1.2 kb HindIII fragment of pMK4 was cloned into the larger pUCTZ37 fragment to construct plasmid pUCTZ47 (Fig. 2). The recombinant plasmid pUCTZ47 contains the full length sequence of nprM and its transcriptional promotor sequence.

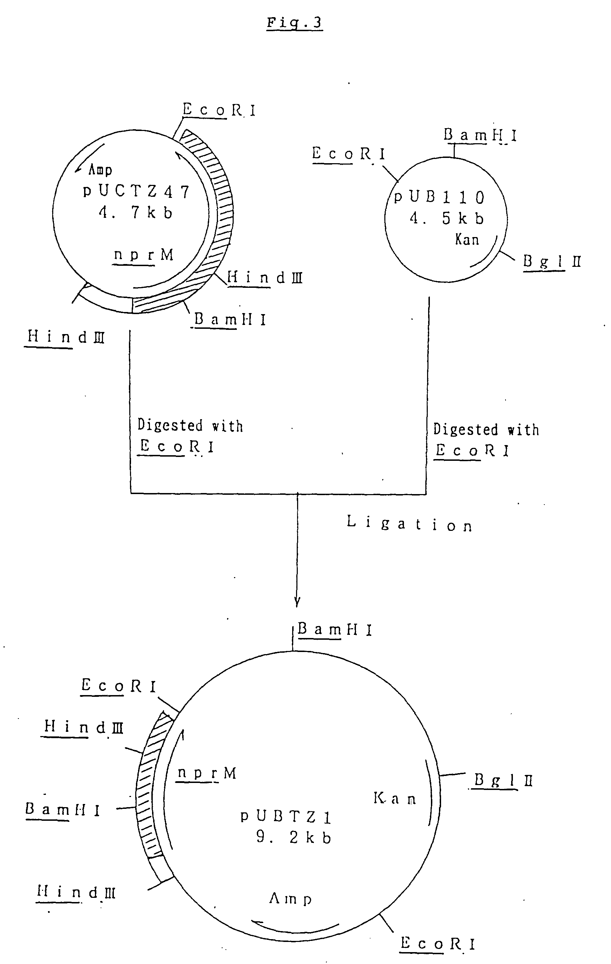

To construct shuttle vectors between Escherichia coli and Bacillus subtilis, both pUCTZ47 and pUB110 (Lacey et al., 1974) were digested with EcoRI and ligated with T4 polynucleotide kinase to construct plasmid pUBTZl, as shown in Fig. 3.

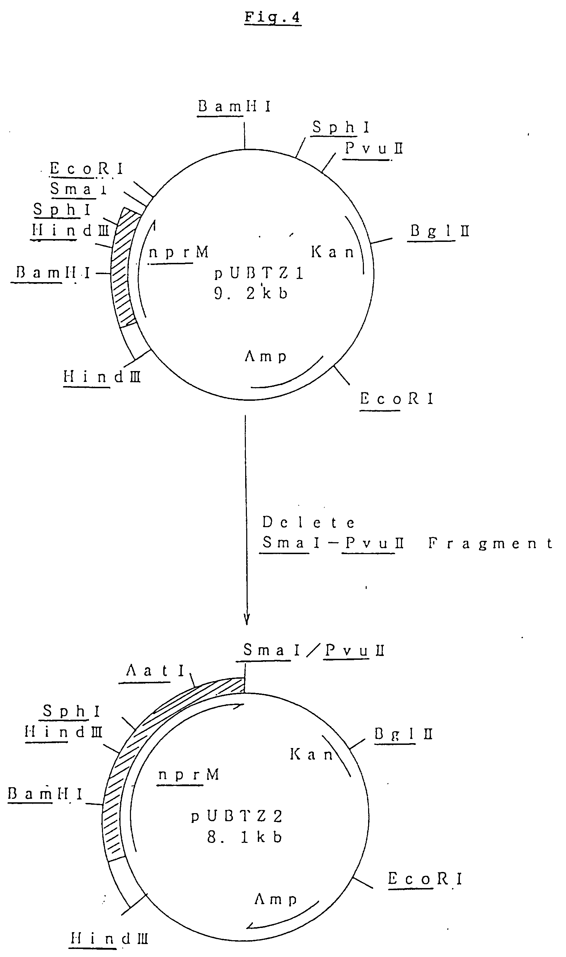

Finally, the DNA fragment between the restriction site by endonuclease SmaI and PvuII was deleted from the plasmid pUBTZ1 as shown in Fig. 4, to construct plasmid pUBTZ2. Plasmid pUBTZ2 has single BamHI, SphI and AatI restriction sites in the nprM gene. The replacement of the wild type gene with the mutant gene was carried out as described below.

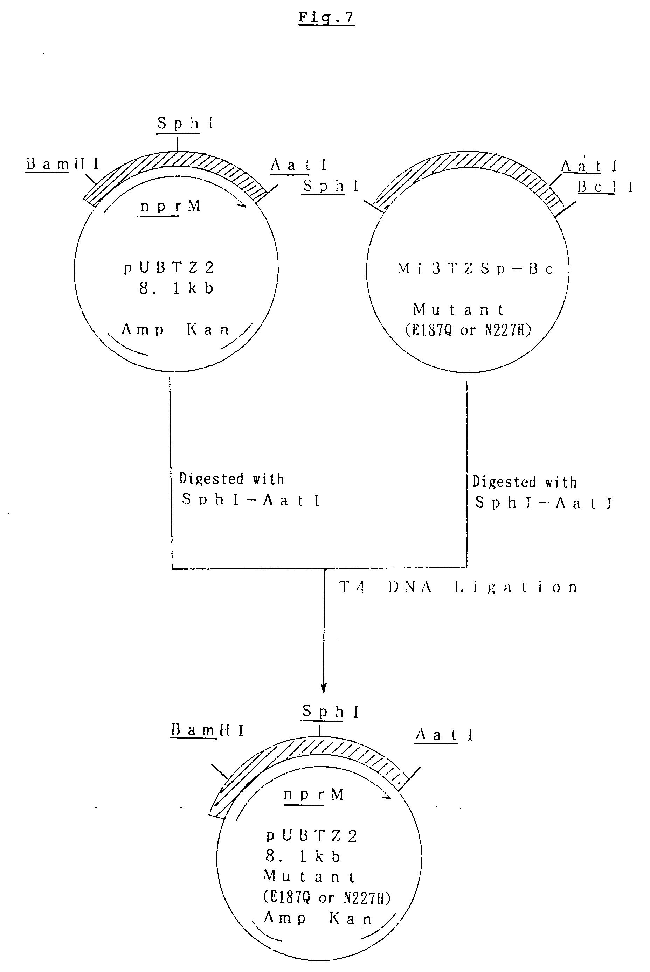

The plasmid pUBTZ2 was digested with Sphl and AatI, and a 7.6 kb fragment was isolated. The mutated SphI-AatI fragments of the nprM gene (about 550 bp) were ligated with the thus-obtained pUBTZ2 SphI-AatI fragment using a Takara Shuzo DNA ligation kit. The ligation mixture was used to transform Escherichia coli JM103 in a conventional manner to give recombinant plasmids pUBTZ2 (E187Q) and pUBTZ2 (N227H) (Fig. 7).

The recombinant plasmid pUBTZ2 (mutant) was transformed to Bacillus subtilis strain MT-2 by a conventional method. The cell suspension was spread on an LB agar plate containing 1% casein and 5 µg/ml kanamycin and incubated at 37°C overnight. Finally the recombinant plasmid pUBTZ2 (mutant) was obtained by isolation of halo-forming colonies.

A single colony of the recombinant Bacillus subtilis MT-2/pUBTZ2 (mutant) was transferred to 5 ml of LB medium containing kanamycin (5 µg/ml) and incubated at 37°C overnight. The culture was transferred to 500 ml of 2L medium (2% Bacto tryptone, 1% yeast extract, 0.5% NaCl) containing kanamycin (5 µg/ml) and incubated at 37°C for 20 hours. The culture broth was centrifuged at 8,000 rpm for 30 minutes to remove bacteria, ammonium sulfate was added to the supernatant to attain 60% saturation and the mixture was stirred overnight at 4°C.

The precipitate was recovered by centrifugation and dissolved in 10 ml of Buffer A (20 mM Tris-hydrochloride at pH 9.0, 10 mM CaCl₂). The solution was applied to 20 ml of Butyl-Toyopearl, followed by an elution with Buffer A at a flow rate of 1.5 ml/minute. Active fractions were combined and subjected to salting out with 60% saturated ammonium sulfate. The precipitate was collected by centrifugation at 15,000 rpm. for 30 minutes, and dissolved in 5 ml of Buffer B (20 mM Tris-hydrochloride at pH 7.5, 10 mM CaCl₂). The enzyme solution was further applied to a gel-filtration column (TSK Gel G2000 SW 21.5 x 600 mm), followed by elution with Buffer B at a flow rate of 1 ml/minute. Active eluate fractions were combined to give the purified enzyme.

### EXAMPLE 2

### (Synthesis of the mutant thermolysin-like neutral metallo-protease which has the mutation at the 144th amino acid residue by random mutagenesis.)

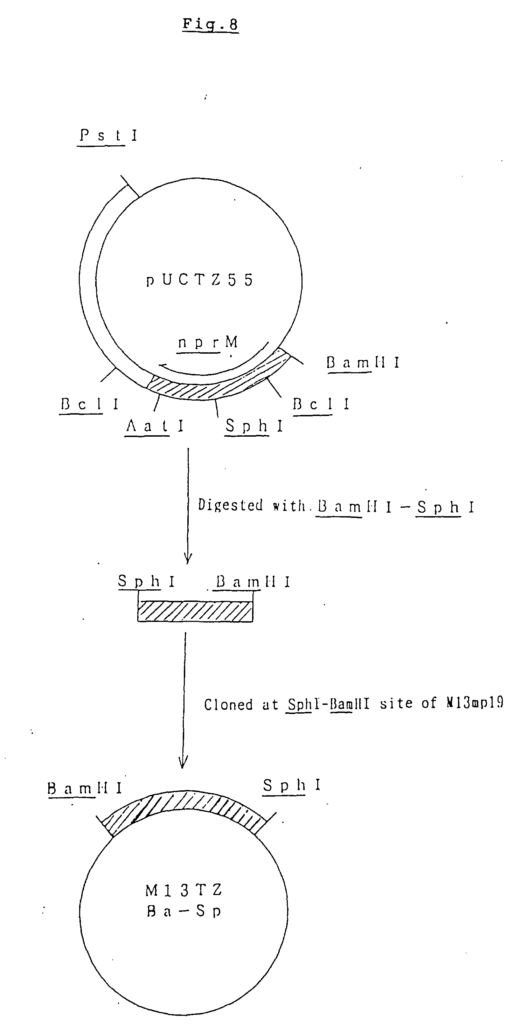

A recombinant phage M13TZBa-Sp containing the BamHI-SphI fragment of the nprM gene was obtained in the same way as described in example 1, except for the restriction enzymes (BamHI and SphI instead of PstI and BamHI) and the M13 phage vector (mpl9 instead of mpl8) used. The procedure for the production of the recombinant phage M13TZBa-Sp is shown in Fig. 8.

The single-stranded DNA of M13TZBa-Sp was incubated at 21.5°C for 4.5 hours in 20 µl of a 0.25 M sodium acetate buffer containing 1M sodium nitrite (pH 4.3), and the DNA was recovered by ethanol precipitation.

The recovered DNA was dissolved in the reaction mixture for PCR (67 mM Tris-hydrochloride pH 8.8, 16.6 mM ammonium sulfate, 6.7 mM MgCl₂, 10 mM 2-mercaptoethanol, 0.2 mM dATP, 0.2 mM dTTIP, 0.2 mM dGTP, 0.2 mM dCTP, 1 µM M13 forward universal primer, 1 µM M13 reverse primer), and 1 unit of Tth DNA polymerase was added. The solution was covered with one drop of mineral oil. The denaturation at 93°C for 1 minute, the annealing at 45°C for 1 minute and the extension at 72°C for 45 seconds were repeated 30 times. After the reaction, the water layer was recovered, extracted with phenol and treated with ethanol to recover the amplified DNA by precipitation.

Half of the quantity of the DNA was digested with 5 units of each of BamHI and SphI at 37°C for 2 hours in 20 µl of a reaction mixture (50 mM Tris-hydrochloride at pH 7.5, 10 mM MgCl₂, 100 mM NaCl, 1 mM DTT). Then the reaction mixture was incubated at 70°C for 5 minutes. The mutated BamHI-SphI fragment was ligated with the BamHI-SphI fragment of pUBTZ2 (7.4 kb) using a Takara Shuzo DNA ligation kit. The ligation mixture was used to transform Escherichia coli JM103 in a conventional method to give transformant JM103/pUBTZ2 (mutant).

3 ml of an alkaline solution (0.2 N NaOH, 0.2% SDS) was added to an agar plate containing the transformant colonies to dissolve the colonies and 1 ml of solution was recovered. 1 ml of 5 M potassium acetate was added to the solution, and the mixture was centrifuged for 20 minutes to remove the precipitate after keeping on ice for 10 minutes. The supernatant was treated with phenol and precipitation was achieved by treatment with ethanol. The precipitate was dissolved after drying in vacuum and used as a mutant library.

The Bacillus subtilis strain MT-2 was transformed by the mutant library in a conventional method. The cell suspension was spread on an LB agar plate containing 1% casein and 5 µg/ml kanamycin and incubated overnight at 37°C and finally 140 halo-forming colonies were isolated.

The single colonies were transferred to 3 ml of LB medium containing 5 µg/ml kanamycin and incubated at 37°C overnight. The culture was centrifuged for 5 minutes at 10,000 rpm. and 0.3 ml of saturated solution of ammonium sulfate and 0.04 ml of butyl-toyopearl 650S was added to 1.2 ml of the supernatant. The mixture was stirred and was kept at room temperature for 5 minutes. Then, the solution was centrifuged at 10,000 rpm for 1 minute to remove the supernatant and the precipitate was suspended in washing solution (8 mM Tris-hydrochloride at pH 8.0, 8 mM calcium chloride, 20% saturated ammonium sulfate) and was packed in a small diameter pipette-type column. After washing with 0.6 ml of washing solution, the enzyme was eluted by using 0.2 ml of eluent (10 mM Tris-hydrochloride at pH 8.0, 10 mM calcium chloride). The enzyme solution was desalted by using gel filtration (Sephadex PD10) and finally 0.5 ml of the purified enzyme solution was obtained.

The concentration of the enzyme was determined by the dye-binding assay (Bradford, M.M., (1976) Anal. Biochem., 72, 248-254) and Z-APM digestion activity was measured for screening of mutated enzymes in the following way.

1 ml of a substrate solution (10 mM Tris-maleate at pH 8.0, 10 mM calcium chloride, 10 mM Z-APM) was mixed with 0.05 ml of the enzyme solution and was incubated at 37°C for 20 minutes. Then 0.5 ml of stop solution (0.1 M acetic acid) was added, followed by further addition of 1 ml of ninhydrin solution. After 15 minutes of incubation at 100°C, 2 ml of 50% ethanol was added and the absorbance was measured at 570 nm. The specific activity defined as the change in absorbance per mg of enzyme protein was expressed in terms of a relative value compared to that of the wild type enzyme. Two clones having higher activity for Z-APM digestion were obtained.

| Enzyme | Z-APM digestion activity (%) |
|---|---|
| Wild type | 100 |
| Clone 1 | 260 |
| Clone 2 | 280 |

It should be noted that these activity values were determined by rough method.

The plasmid DNA was extracted from these two clones and the nucleotide sequences of these clones were determined. It was found from the nucleotide sequences that the two clones were identical, and that the 38th glycine residue and the 144th leucine residue had been replaced by glutamine and serine respectively. The clone is represented as G38Q-L144S hereafter.

1 µg of each of the plasmid pUBTZ2(G38Q-L144S) and pUI3TZ2(wild type) were incubated in 20 µl of a reaction mixture (50 mM Tris-hydrochloride at pH 7.5, 10 mM magnesium chloride, 50 mM sodium chloride, 1 mM DTT) containing 5 units of HindIII at 37°C for 2 hours. These samples were electrophoresed on an 1% agarose gel to isolate an about 1.1 kb DNA fragment and an about 7 kb DNA fragment respectively, and both fragments were purified using BIO-101 Gene Clean DNA purification kit.

The DNA fragment of about 7 kb derived from pUBTZ2(G38Q-Ll44S) and the DNA fragment of about 1.1 kb derived from pUBTZ2(wild type) were ligated using Takara Shuzo DNA ligation kit. Each ligation mixture was used to transform Escherichia coli JM103 in a conventional method. The transformants were screened by rapid DNA isolation and DNA sequencing to isolate the clone of pUBTZ2(L144S).

Each single colony of each transformant Bacillus subtilis MT-2/pUBTZ2(G38Q-L144S) and MT-2/pUBTZ2(L144S) was transferred to 5 ml of LB medium containing kanamycin (5 µg/ml) at 37°C overnight. The culture was transferred to 500 ml of 2L medium (2% Bacto tryptone, 1% yeast extract, 0.5% NaCl) containing kanamycin (5 µg/ml) and incubated at 37°C for 20 hours. The culture broth was centrifuged at 8,000 rpm for 30 minutes to remove bacteria, ammonium sulfate was added to the supernatant to attain 60% saturation and the mixture was stirred overnight at 4°C.

The precipitate was recovered by centrifugation and dissolved in 10 ml of Buffer A (20 mM Tris-hydrochloride at pH 9.0, 10 mM CaCl₂). The solution was applied to 20 ml of Butyl-Toyopearl, followed by an elution with Buffer A at a flow rate of 1.5 ml/minute. Active fractions were combined and subjected to salting out with 60% saturated ammonium sulfate. The precipitate was collected by centrifugation at 15,000 rpm for 30 minutes, and dissolved in 5 ml of Buffer B (20 mM Tris-hydrochloride at pH 7.5, 10 mM CaCl₂). The enzyme solution was further applied to a gel-filtration column (TSK Gel G2000 SW 21.5 x 600 mm), followed by elution with Buffer B at a flow rate of 1 ml/minute. Active fractions were combined to give each purified enzyme.

### EXAMPLE 3

### (Synthesis of the mutant thermolysin-like neutral metallo-protease which has the mutation at the 150th amino acid residue)

The oligonucleotides used for the mutagenesis were synthesized by using an DNA synthesizer Model 380B produced by Applied Biosystems Co. LTD. The nucleotide sequences of the mutagenesis primers were

Furthermore, a reverse-direction primer was synthesized having the nucleotide sequence described below. 1 ng of plasmid pUBTZ2 was dissolved in 100 µl of the reaction mixture for PCR (67 mM Tris-hydrochloride (pH 8.8), 16.6 mM ammonium sulfate, 6.7 mM MgCl₂, 10 mM 2-mercaptoethanol, 0.05 mM dATP, 0.05 mM dTTP, 0.05 mM dGTP, 0.05 mM dCTP, 1 µM mutagenesis primer, 1 µM reverse-direction primer), and 1 unit of Tth DNA polymerase was added. The solution was covered with one drop of mineral oil. The denaturation at 93°C for 1 minute, the annealing at 45°C for 1 minute and the extension at 72°C for 45 seconds were repeated 30 times. After the reaction, the water layer was recovered, extracted with phenol and treated with ethanol to recover the amplified DNA.

20 µl of a reaction mixture (50 mM Tris-hydrochloride at pH 7.5, 10 mM MgCl₂, 100 mM ·NaCl, 1 mM DTT) containing half amount of the DNA was digested with 5 units each of SphI and AatI at 37°C for 2 hours, and was incubated at 70°C for 5 minutes. The mutated SphI-AatI fragment was ligated with SphI-AatI fragment of pUBTZ2 (7.6 kb) using a Takara Shuzo DNA ligation kit. The ligation mixture was used to transform Escherichia coli JM103 in a conventional method to give a transformant JM103/pUBTZ2 (mutant). The substituted amino acid was confirmed by the determination of the nucleotide sequence of these plasmid.

A single colony of the transformant Bacillus subtilis MT-2/pUBTZ2(mutant) was transferred to 5 ml of LB medium containing kanamycin (5 µg/ml) at 37°C overnight. The culture was transferred to 500 ml of 2L medium (2% Bacto tryptone, 1% yeast extract, 0.5% NaCl) containing kanamycin (5 µg/ml) and incubated at 37°C for 20 hours. The culture broth was centrifuged at 8,000 rpm for 30 minutes to remove bacteria, ammonium sulfate was added to the supernatant to attain 60% saturation and the mixture was stirred overnight at 4°C.

The precipitate was recovered by centrifugation and dissolved in 10 ml of Buffer A (20 mM Tris-hydrochloride at pH 9.0, 10 mM CaCl₂). The solution was applied to 20 ml of Butyl-Toyopearl, followed by an elution with Buffer A at a flow rate of 1.5 ml/minute. Active fractions were combined and subjected to salting out with 60% saturated ammonium sulfate. The precipitate was collected by centrifugation at 15,000 rpm for 30 minutes, and dissolved in 5 ml of Buffer B (20 mM Tris-hydrochloride at pH 7.5, 10 mM CaCl₂). The enzyme solution was further applied to a gel-filtration column (TSK Gel G2000 SW 21.5 x 600 mm), followed by elution with Buffer B at a flow rate of 1 ml/minute. Active fractions were combined to give each purified enzyme.

### EXAMPLE 4

### (Synthesis of a mutant thermolysin-like neutral metalloprotease which has mutations at the 144th, 150th and 227th amino acid residues)

A three-site mutants of thermolysin-like neutral metalloprotease was constructed as follows. The plasmid pUBTZ2 (N227H), which represents the plasmid containing the mutation at the codon of the 227th amino acid residue from asparagine to histidine, was used as a template for polymerase chain reaction. The mutagenesis primers and the revers-direction primer were the same as those described in Example 3.
The mutagenesis primers: The reverse-direction primer:

1 ng of plasmid pUBTZ2 (N227H) was dissolved in 100 µl of the reaction mixture of PCR (67 mM Tris-hydrochloride (pH 8.8), 16.6 mM ammonium sulfate, 6.7 mM MgCl₂, 10 mM 2-mercaptoethanol, 0.05 mM dATP, 0.05 mM dTTP, 0.05 mM dGTP, 0.05 mM dCTP, 1 µM mutagenesis primer, 1 µM reverse-direction primer), 1 unit of Tth DNA polymerase was added. The solution was covered with one drop of mineral oil. The denaturation at 93°C for 1 minute, the anneal at 45°C for 1 minute and the extension at 72°C for 45 seconds were repeated 30 times. After the reaction, the water layer was recovered, extracted with phenol and precipitated in ethanol to recover the amplified DNA (D150N-N227H (Example 4A), D150H-N227H (Example 4B)).

20 µl of reaction mixture (50 mM Tris-hydrochloride at pH 7.5, 10 mM MgCl₂, 100 mM NaCl, 1mM DTT) containing half the quantity of the DNA was digested with 5 units of each SphI and AatI at 37 °C for 2 hours, and was treated at 70°C for 5 minutes. The mutated SphI-AatI fragment was ligated with the 7.6 kb SphI-AatI fragment of pUBTZ2 (L144S) using a Takara Shuzo DNA ligation kit. The ligation mixture was used to transform Eschericha coli JM103 in a conventional method to give a transformant JM103/pUBTZ2 (mutant). The substituted amino acid was confirmed by the determination of the nucleotide sequence of these plasmid.

A single colony of the transformant Bacillus subtilis MT-2/pUBTZ2 (mutant) was inoculated in 5 ml of LB medium containing kanamycin (5 µg/ml) at 37°C overnight. The culture was transferred to 500 ml of 2L medium (2 % Bacto tryptone, 1 % yeast extract, 0.5 % NaCl) containing kanamycin (5 µg/ml) and incubated at 37°C for 20 hours. The culture broth was centrifuged at 8,000 rpm for 30 minutes to remove bacteria, ammonium sulfate was added to the supernatant to attain 60 % saturation and the mixture was stirred overnight at 4°C.

The precipitate was recovered by centrifugation and dissolved in 10 ml of the buffer (20 mM Tris-hydrochloride at pH 9.0, 10 mM CaCl₂). The solution was applied to 20 ml of Butyl-Toyopearl, followed by elution with the same buffer at a flow rate of 1.5 ml/minutes. Active fractions were combined and subjected to salting out with 60 % % saturated ammonium sulfate. The precipitate was collected by centrifugation at 15,000 rpm. for 30 minutes, and dissolved in 5 ml of the buffer (20 mM Tris-hydrochloride at pH 7.5, 10 mM CaCl₂). The enzyme solution was further applied to a gel-filtration column (TSK Gel G2000 SW 21.5 x 600 mm), followed by elution with the same buffer at a flow rate of 1 ml/minutes. Active fractions were combined to give each purified enzyme (L144S-D150N-N227H (Example 4A), L144S-D150H-N227H (Example 4B)).

Two recombinant Bacillus subtilis strains which express the modified thermolysin-like neutral metallo-proteases according to the invention, namely, Bacillus subtilis MT-2/pUBTZ2(TZ-1) wherein the 144th leucine residue is replaced by serine, the 150th aspartic acid residue is replaced by histidine and the 227th asparagine residue is replaced by histidine, and Bacillus subtilis MT-2/pUBTZ2(TZ-2) wherein the 144th leucine residue was replaced by serine, the 150th aspartic acid residue was replaced by asparagine and the 227th asparagine residue was replaced by histidine, have been deposited at the Fermentation Research Institute, Agency of Industrial Science and Technology, Ministry of International Trade and Industry, Japan (1-3, Higashi 1-chome, Tsukuba-shi, Ibaraki, 305 Japan) under the deposition Nos. FERM BP-4112 and FERM BP-4113, respectively.

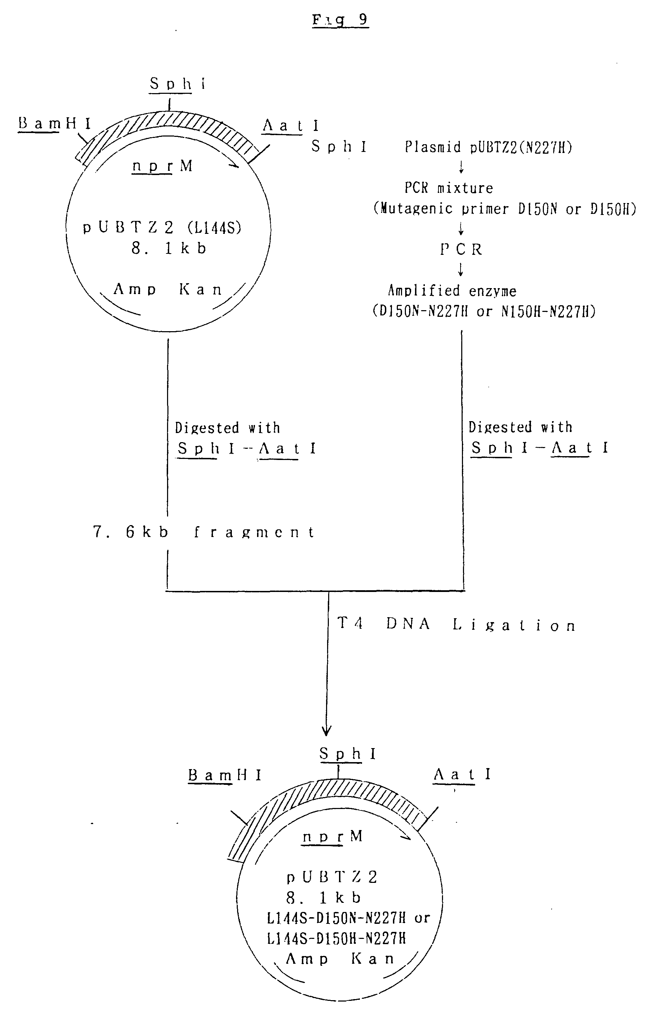

Fig. 9 shows the scheme used for constructing recombinant plasmid pUBTZ2 (mutant) (L144S-D150N-N227H or L144S-D150H-N227H).

### EXAMPLE 5

### (Determination of the activity for casein digestion and Z-APM digestion)

Various modified enzymes such as N227H which represents the enzyme containing a histidine at the 227th amino acid residue (originally asparagine), N227K which represents the enzyme containing a lysine at the 227th amino acid residue (originally asparagine), N227R which represents the enzyme containing an arginine at the 227th amino acid residue (originally asparagine), L144S which represents the enzyme containing a serine at the 144th amino acid residue (originally leucine), E187Q which represents the enzyme containing a glutamine at the 187th amino acid residue (originally glutamic acid), D150N which represents the enzyme containing an asparagine at the 150th amino acid residue (originally aspartic acid), D150H which represents the enzyme containing a histidine at the 150th amino acid residue (originally aspartic acid), L144S-D150N-N227H which represents the enzyme containing a serine at the 144th position (originally leucine), an asparagine at the 150th position (originally aspartic acid), and a histidine at the 227th amino acid residue (originally asparagine) and L144S-D150H-N227H which represents the enzyme containing a serine at the 144th position (originally leucine), a histidine at the 150th position (originally aspartic acid) and a histidine at the 227th amino acid residue (originally asparagine) were used to determine the activity of casein digestion and that of Z-APM digestion.

For casein digestion method, 1 ml of each purified enzyme solution (prepared by dissolving the enzyme in 10 mM borate buffer at pH 8.0, 2 mM calcium sulfate) was added to 1 ml of substrate solution (1% casein, 1/15 M phosphate buffer at pH 7.2). The mixture was incubated at 35°C for 10 minutes, then 2.0 ml of stop solution (0.1 M TCA, 0.2 M sodium acetate, 0.3 M acetic acid) were added, followed by further incubation at 35°C for 30 minutes. After filtration, 5 ml of alkaline solution (0.4 M sodium carbonate) and 1 ml of Folin reagent (5 times dilution of phenol reagent with distilled water) were added to 1 ml of filtrate and incubated at 35°C for 20 minutes. The absorbance of the mixture at 660 nm was measured. One unit (PU) of the enzyme liberates from milk casein an amount of the digestion product which is capable of giving Folin's color (as determined by increase of the absorbance at 660 nm) equivalent to 1 µg of tyrosine per minute of the early stage during the reaction at 35°C, pH 7.2. Specific activity (PU/mg) was compared to wild type enzyme and the relative values are given in Table 1.

For Z-APM digestion method, 0.5 ml of each purified enzyme solution (prepared by dissolving the enzyme in 10 mM Tris-maleate buffer at pH 8.0, 10 mM calcium chloride) was added to 0.5 ml of a substrate solution (10 mM Tris-maleate at pH 8.0, 10 mM calcium chloride, 10 mM Z-APM). The mixture was incubated at 37°C for 20 minutes, then 0.5 ml of stop solution (0.1 M acetic acid) was added, followed by further addition of 1 ml of ninhydrin solution. After 15 minutes of treatment at 100°C, 2 ml of 50% ethanol was added and the absorbance was measured at 570 nm. The specific activity defined as the change in absorbance per mg of enzyme was expressed in terms of a relative value compared to that of the wild type enzyme. The results are shown in Table 1.

**TABLE 1**

| Enzyme | Activity for casein digestion (%) | Activity for Z-APM digestion (%) |
|---|---|---|
| N227H | 90 | 160 |
| N227K | 115 | 150 |
| N227R | 110 | 200 |
| L144S | 42 | 190 |
| E187Q | 100 | 150 |
| D150N | 120 | 130 |
| D150H | 92 | 140 |
| L144S-N227H | 41 | 300 |
| L144S-D150N | 22 | 152 |
| L144S-D150H | 29 | 184 |
| D150N-N227H | 116 | 197 |
| D150H-N227H | 107 | 324 |
| L144S-D150H-N227H (TZ-1) | 35 | 500 |
| L144S-D150N-N227H (TZ-2) | 32 | 430 |
| Wild type | 100 | 100 |

### EXAMPLE 6

### (Z-APM synthesising activity)

Various modified enzymes such as N227H which represents the enzyme containing a histidine at the 227th amino acid residue (originally asparagine), L144S which represents the enzyme containing a serine at the 144th amino acid residue (originally leucine), D150N which represents the enzyme containing an asparagine at the 150th amino acid residue (originally aspartic acid), D150H which represents the enzyme containing a histidine at the 150th amino acid residue (originally aspartic acid), L144S-D150N-N227H which represents the enzyme containing a serine at the 144th position (originally leucine), an asparagine at the 150th position (originally aspartic acid), and a histidine at the 227th amino acid residue (originally asparagine) and L144S-D150H-N227H which represents the enzyme containing a serine at the 144th position (originally leucine), a histidine at the 150th position (originally aspartic acid), and a histidine at the 227th amino acid residue (originally asparagine) were used to determine the Z-APM synthesizing activity.

0.1 ml of each purified enzyme solution (1 mg/ml) was added to 1 ml of a substrate solution (0.1 M benzyloxycarbonyl-α-L-aspartic acid, 0.1 M L-phenylalanine methyl ester hydrochloride and 0.2 M Tris-maleate buffer at pH 7.0) and, after mixing, the enzymatic reaction was carried out (in a water bath) at 37°C. After 10, 20 and 30 minutes, 10 µl of each reaction mixture was sampled and analyzed by HPLC with a reverse phase column. The quantity of Z-APM produced by the enzymatic reaction was calculated, using a standard sample of Z-APM as a reference, and the specific activity per mg of enzyme was expressed in terms of relative values based on that of the wild type enzyme. The results are shown in Table 2.

**TABLE 2**

| Enzyme | Z-APM synthesizing activity (%) |
|---|---|
| N227H | 150 |
| L144S | 150 |
| D150N | 150 |
| D150H | 200 |
| L144S-N227H | 181 |
| L144S-D150N | 270 |
| L144S-D150H | 236 |
| D150N-N227H | 266 |
| D150H-N227H | 350 |
| L144S-D150H-N227H (TZ-1) | 500 |
| L144S-D150N-N227H (TZ-2) | 400 |
| Wild type | 100 |

### EXAMPLE 7

### (pH Effects on the synthesis of Z-APM of mutant enzymes)

The effect of pH on the synthesis of Z-APM were determined for both the mutant enzymes (L144S-D150H-N227H (TZ-1), L144S-D150N-N227H (TZ-2)) and wild type thermolysin-like neutral metallo-protease. The measurements were made in the same way as Example 6 except for the pH of Tris-maleate buffer (pH 5.0-8.0).

The results are shown in Table 3. The activity was represented as a relative value against the wild type enzyme at pH 7.0. The activity of Z-APM synthesis appears to be greatly influenced by the pH in the reaction mixture.

It is preferable to synthesize Z-APM at lower pH, because at lower pH less hydrolysis of the methyl ester of the substrates occurs. The present invention presents a solution to such problems in that higher enzyme activities are found at lower pH than for the wild type enzyme at its optimum pH. The activities of L144S-D150H-N227H (TZ-1) and L144S-D150N-N227H (TZ-2) at pH 6.0 are much higher than that of wild type enzyme at pH 7.0.

**TABLE 3**

| | Z-APM synthesizing activity (%) | | | |
|---|---|---|---|---|
| | pH value | | | |
| Enzyme | 5.0 | 6.0 | 7.0 | 8.0 |
| L144S-D150H-N227H (TZ-1) | 23 | 274 | 517 | 154 |
| L144S-D150N-N227H (TZ-2) | 38 | 234 | 391 | 140 |
| Wild type | 10 | 56 | 100 | 49 |

### BRIEF DESCRIPTION OF THE DRAWINGS

Fig. 1 shows the scheme used for constructing a recombinant plasmid named pUCTZ37 from the known plasmid pMK4.

Fig. 2 shows the scheme used for constructing a recombinant plasmid named pUCTZ47 from the plasmid pMK4 and the plasmid pUCTZ37.

Fig. 3 shows the scheme used for constructing a recombinant plasmid named pUBTZ1 from the known plasmid pUCTZ47 and the plasmid pUB110.

Fig. 4 shows the scheme used for constructing a plasmid named pUBTZ2 from the plasmid pUBTZ1.

Fig. 5 shows the scheme used for constructing a recombinant plasmid named pUCTZ55 from the known plasmid pMK1.

Fig. 6 shows the scheme used for constructing a recombinant M13 phage named M13TZSp-Bc from the plasmid pUCTZ55.

Fig. 7 shows the scheme used for constructing a plasmid pUBTZ2 (mutant) from the plasmid pUBTZ2 and M13TZSp-Bc (mutant).

Fig. 8 shows the scheme used for constructing a recombinant M13 phage named M13TZBa-Sp from the plasmid pUCTZ55.

Fig. 9 shows the scheme used for constructing recombinant plasmid pUBTZ2 (mutant) (L144S-D150N-N227H or L144S-D150H-N227H) from the plasmid pUBTZ2 (L144S) and the mutagenic PCR products obtained from the plasmid pUBTZ2 (N227H) and the reaction mixture of PCR containing the mutagenesis primers (D150N).

While the invention has been described in detail and with reference to specific examples thereof, it will be apparent to one skilled in the art that various changes can be made therein without departing from the spirits and scope thereof.

## Claims

1. A modified protease of thermolysin-like neutral metallo-protease having the amino acid sequence of SEQ ID NO:1, wherein at least one amino acid residue selected from the group consisting of the 144th leucine residue, 150th aspartic acid residue, the 187th glutamic acid residue and the 227th asparagine residue is replaced with an amino acid residue other than said amino acid residue and having an improved activity in the synthesis of benzyloxycarbonyl-α-L-aspartyl-L-phenylalamine methyl ester in comparison with the wild-type metallo-protease having the amino acid sequence of SEQ ID NO:1.

2. A modified protease of claim 1, wherein the 144th leucine residue is replaced with a serine.

3. A modified protease of claim 1, wherein the 150th aspartic acid residue is replaced with an asparagine, histidine or lysine residue.

4. A modified protease of claim 1, wherein the 187th glutamic acid residue is replaced with an glutamine.

5. A modified protease of claim 1, wherein the 227th asparagine residue is replaced with an histidine, lysine or arginine.

6. A modified protease of claim 1, wherein the 144th- leucine residue is replaced with a serine and the 150th aspartic acid residue is replaced with a histidine and the 227th asparagine residue is replaced with a histidine.

7. A modified protease of claim 1, wherein the 144th leucine residue is replaced with a serine and the 150th aspartic acid residue is replaced with a asparagine and the 227th asparagine residue is replaced with a histidine.

8. Use of the modified protease of any of claims 1 to 7 for the digestion or the synthesis of benzyloxycarbonyl-α-L-aspartyl-L-phenylalanine methyl ester.

9. A process for synthesizing benzyloxy-carbonyl-α-L-aspartyl-L-phenylalanine methyl ester which comprises contacting the modified protease of any of claims 1 to 7 with a substrate solution containing benzyloxycarbonyl-α-L-aspartic acid and L- or D,L-phenylalanine methyl ester.

10. A process for digesting benzyloxycarbonyl-α-L-aspartyl-L-phenylalanine methyl ester which comprises contacting the modified protease of any of claims 1 to 7 with a substrate solution containing benzyloxycarbonyl-α-L-aspartyl-L-phenylalanine methyl ester.

## Patentansprüche

1. Modifizierte Protease einer Thermolysin-artigen, neutralen Metallprotease mit der Aminosäuresequenz SEQ ID NO:1, worin mindestens ein Aminosäurerest ausgewählt aus der Gruppe bestehend aus dem 144. Leucin-Rest, dem 150. Asparaginsäure-Rest, dem 187. Glutaminsäure-Rest und dem 227. Asparagin-Rest ersetzt ist durch einen anderen als diesen Aminosäurerest, und mit einer verbesserten Aktivität bei der Synthese von Benzyloxycarbonyl-α-L-aspartyl-L-phenylalanin-methylester im Vergleich zur Wildtyp-Metallprotease mit der Aminosäuresequenz SEQ ID NO:1.

2. Modifizierte Protease nach Anspruch 1, worin der 144. Leucin-Rest durch ein Serin ersetzt ist.

3. Modifizierte Protease nach Anspruch 1, worin der 150. Asparaginsäure-Rest durch einen Asparagin-, Histidin- oder Lysin-Rest ersetzt ist.

4. Modifizierte Protease nach Anspruch 1, worin der 187. Glutaminsäure-Rest duch ein Glutamin ersetzt ist.

5. Modifizierte Protease nach Anspruch 1, worin der 227. Asparagin-Rest durch ein Histidin, Lysin oder Arginin ersetzt ist.

6. Modifizierte Protease nach Anspruch 1, worin der 144. Leucin-Rest durch ein Serin ersetzt ist und der 150. Asparaginsäure-Rest durch ein Histidin ersetzt ist und der 227. Asparagin-Rest durch ein Histidin ersetzt ist.

7. Modifizierte Protease nach Anspruch 1, worin der 144. Leucin-Rest durch ein Serin ersetzt ist und der 150. Asparaginsäure-Rest durch ein Asparagin ersetzt ist und der 227. Asparagin-Rest durch ein Histidin ersetzt ist.

8. Verwendung der modifizierten Protease nach einem der Ansprüche 1 bis 7 für den Verdau oder die Synthese von Benzyloxycarbonyl-a-L-aspartyl-L-phenylalanin-methylester.

9. Verfahren zur Synthetisierung von Benzyloxycarbonyl-α-L-aspartyl-L-phenylalanin-methylester, welches das Kontaktieren der modifizierten Protease nach einem der Ansprüche 1 bis 7 mit einer Substratlösung, die Benzyloxycarbonyl-α-L-asparaginsäure und Loder D,L-Phenylalaninmethylester enthält, umfasst.

10. Verfahren zum Verdau von Benzyloxycarbonyl-α-L-aspartyl-L-phenylalanin-methylester, welches das Kontaktieren der modifizierten Protease nach einem der Ansprüche 1 bis 7 mit einer Substratlösung, die Benzyloxycarbonyl-α-L-aspartyl-L-phenylalaninmethylester enthält, umfasst.

## Revendications

1. Protéase modifiée à partir de la métalloprotéase neutre ressemblant à la thermolysine, ayant la séquence d'aminoacides de SEQ ID n° 1, dans laquelle au moins un résidu aminoacide, choisi dans l'ensemble constitué par le 144^{e} résidu leucine, le 150^{e} résidu acide aspartique, le 187^{e} résidu acide glutamique et le 227^{e} résidu asparagine, est remplacé par un résidu aminoacide autre que ledit résidu aminoacide, et ayant une activité améliorée dans la synthèse de l'ester méthylique de benzyloxycarbonyl-α-L-aspartyl-L-phénylalanine par comparaison avec la métalloprotéase de type sauvage ayant la séquence d'aminoacides de SEQ ID n° 1.

2. Protéase modifiée de la revendication 1, dans laquelle le 144^{e} résidu leucine est remplacé par une sérine.

3. Protéase modifiée de la revendication 1, dans laquelle le 150^{e} résidu acide aspartique est remplacé par un résidu asparagine, histidine ou lysine.

4. Protéase modifiée de la revendication 1, dans laquelle le 187^{e} résidu acide glutamique est remplacé par une glutamine.

5. Protéase modifiée de la revendication 1, dans laquelle le 227^{e} résidu asparagine est remplacé par une histidine, lysine ou arginine.

6. Protéase modifiée de la revendication 1, dans laquelle le 144^{e} résidu leucine est remplacé par une sérine et le 150^{e} résidu acide aspartique est remplacé par une histidine, et le 227^{e} résidu asparagine est remplacé par une histidine.

7. Protéase modifiée de la revendication 1, dans laquelle le 144^{e} résidu leucine est remplacé par une sérine et le 150^{e} résidu acide aspartique est remplacé par une asparagine, et le 227^{e} résidu asparagine est remplacé par une histidine.

8. Utilisation de la protéase modifiée de l'une quelconque des revendications 1 à 7, pour la digestion ou la synthèse de l'ester méthylique de benzyloxycarbonyl-α-L-aspartyl-L-phénylalanine.

9. Procédé pour la synthèse de l'ester méthylique de benzyloxycarbonyl-α-L-aspartyl-L-phénylalanine, comprenant la mise en contact de la protéase modifiée de l'une quelconque des revendications 1 à 7, avec une solution de substrat contenant de l'acide benzyloxycarbonyl-α-L-aspartique et de l'ester méthylique de L- ou D,L-phénylalanine.

10. Procédé pour la digestion de l'ester méthylique de benzyloxycarbonyl-α-L-aspartyl-L-phénylalanine, comprenant la mise en contact de la protéase modifiée de l'une quelconque des revendications 1 à 7 avec une solution de substrat contenant de l'ester méthylique de benzyloxycarbonyl-α-L-aspartyl-L-phénylalanine.


### SEQUENCE LISTING

(1) GENERAL INFORMATION:
   (i) APPLICANT:
      (A) NAME: Holland Sweetener Company V.O.F
      (B) STREET: Blekerij 52, 6212 XW
      (C) CITY: Maastricht
      (E) COUNTRY: The Netherlands
      (F) POSTAL CODE (ZIP): 6201
      (G) TELEPHONE: 31 (0)43 21 22 28
      (H) TELEFAX: 31 (0)43 21 66 33
      (I) TELEX: 56 384

      (A) NAME: Sagami Chemical Research Center
      (B) STREET: 4-5, Marunouchi 1-chome, Chiyoda-ku
      (C) CITY: Tokyo
      (E) COUNTRY: Japan
      (F) POSTAL CODE (ZIP): 100
   (ii) TITLE OF INVENTION: NOVEL PROTEASE
   (iii) NUMBER OF SEQUENCES: 10
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 316 amino acids
      (B) TYPE: amino acid
      (D) TOPOLOGY: linear
   (ii) MOLECULE TYPE: protein
   (xi) SEQUENCE DESCRIPTION: SEQ ID NO: 1:
   Ile Thr Gly Thr Ser Thr Val Gly Val Gly Arg Gly Val Leu Gly Asp
   1               5                   10                  15
   Gln Lys Asn Ile Asn Thr Thr Tyr Ser Thr Tyr Tyr Tyr Leu Gln Asp
               20                  25                  30
   Asn Thr Arg Gly Asn Gly Ile Phe Thr Tyr Asp Ala Lys Tyr Arg Thr
           35                  40                  45
   Thr Leu Pro Gly Ser Leu Trp Ala Asp Ala Asp Asn Gln Phe Phe Ala
       50                  55                  60
   Ser Tyr Asp Ala Pro Ala Val Asp Ala His Tyr Tyr Ala Gly Val Thr
   65                  70                  75                  80
   Tyr Asp Tyr Tyr Lys Asn Val His Asn Arg Leu Ser Tyr Asp Gly Asn
                    85                  90                  95
   Asn Ala Ala Ile Arg Ser Ser Val His Tyr Ser Gln Gly Tyr Asn Asn
               100                 105                 110
   Ala Phe Trp Asn Gly Ser Gln Met Val Tyr Gly Asp Gly Asp Gly Gln
           115                 120                 125
   Thr Phe Ile Pro Leu Ser Gly Gly Ile Asp Val Val Ala His Glu Leu
       130                 135                 140
   Thr His Ala Val Thr Asp Tyr Thr Ala Gly Leu Ile Tyr Gln Asn Glu
   145                 150                 155                 160
   Ser Gly Ala Ile Asn Glu Ala Ile Ser Asp Ile Phe Gly Thr Leu Val
                   165                 170                 175
   Glu Phe Tyr Ala Asn Lys Asn Pro Asp Trp Glu Ile Gly Glu Asp Val
               180                 185                 190
   Tyr Thr Pro Gly Ile Ser Gly Asp Ser Leu Arg Ser Met Ser Asp Pro
           195                 200                 205
   Ala Lys Tyr Gly Asp Pro Asp His Tyr Ser Lys Arg Tyr Thr Gly Thr
       210                 215                 220
   Gln Asp Asn Gly Gly Val His Ile Asn Ser Gly Ile Ile Asn Lys Ala
   225                 230                 235                 240
   Ala Tyr Leu Ile Ser Gln Gly Gly Thr His Tyr Gly Val Ser Val Val
                   245                 250                 255
   Gly Ile Gly Arg Asp Lys Leu Gly Lys Ile Phe Tyr Arg Ala Leu Thr
               260                 265                 270
   Gln Tyr Leu Thr Pro Thr Ser Asn Phe Ser Gln Leu Arg Ala Ala Ala
           275                 280                 285
   Val Gln Ser Ala Thr Asp Leu Tyr Gly Ser Thr Ser Gln Glu Val Ala
       290                 295                 300
   Ser Val Lys Gln Ala Phe Asp Ala Val Gly Val Lys
   305                 310                 315
(2) INFORMATION FOR SEQ ID NO: 2:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 2:
   CCAGATTGGC AAATTGGAGA G 21
(2) INFORMATION FOR SEQ ID NO: 3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 3:
   CGCAAGATCA TGGCGGGG 18
(2) INFORMATION FOR SEQ ID NO: 4:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 4:
(2) INFORMATION FOR SEQ ID NO: 5:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 5:
(2) INFORMATION FOR SEQ ID NO: 6:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 6:
(2) INFORMATION FOR SEQ ID NO: 7:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 7:
   GAGATACCAC TTTATTTCAC CCCA 24
(2) INFORMATION FOR SEQ ID NO: 8:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 8:
   AACGATGCGG TAACCAATTT ATACAGC 27
(2) INFORMATION FOR SEQ ID NO: 9:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 9:
   AACGATGCGG TAACCCATTT ATACAGC 27
(2) INFORMATION FOR SEQ ID NO: 10:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: Other nucleic acid (synthetic DNA)
   (xi) SEQUENCE DESCRIPTION: SEQ ID NO: 10:
   GAGATACCAC TTTATTTCAC CCCA                                      24